# EUROPEAN PATENT APPLICATION

(11) **EP 0 850 613 A1**
(43) Date of publication of application: **01.07.1998**
(21) Application number: 97929547.4
(22) Date of filing: 07.07.1997
(51) Int. Cl.: A61F 5/441, A61F 5/445

(54) **EXCREMENT COLLECTING BAG**

(30) Priority: 05.07.1996 JP 195611/96; 08.11.1996 JP 312727/96
(71) Applicant: Alcare Co., Ltd., Sumida-ku, Tokyo 131 (JP)
(72) Inventor: TAKAHASHI, Tetsuya, Haimu Matsuno 201, Tokyo 141 (JP); SANADA, Hiromi, Kanazawa-shi, Ishikawa 920-01 (JP); KONYA, Chizuko, Kanazawa-shi, Ishikawa 920 (JP)
(74) Representative: Preissner, Nicolaus, Dipl.-Ing.
(86) International application number: JP9702348
(87) International publication number: WO9801094

(57) **Abstract**

The body wastes receiving pouch appliance according to the present invention is detachably secured to or over a body wastes discharge hole or opening of a human body, said body wastes receiving pouch appliance comprising a pouch member 5 for receiving and storing body wastes 10, said pouch member having a deodorant-containing body 7, wherein, when the body wastes are taken out for removal from within said pouch member, said deodorant-containing body is broken, whereby the deodorant sealed up in said deodorant-containing body is discharged into said pouch member to thereby prevent the production of the odor.

## Description

### [Technical Field]

The present invention relates to a body wastes receiving pouch appliance for receiving the body wastes discharged from a body wastes discharge hole or opening of a human body.

### [Background Technique]

The urine and feces discharged by incontinence from the natural excretory holes or openings, that is, the urethral meatus and the anus, of a human body, the excrements discharged from the excretory hole or opening such as, e.g. an artificial anus or artifical urinary bladder which has been formed by artificially altering the natural excretory hole or opening due to a disease, or the various body wastes discharged from a fistulous hole or opening which has resulted from a disease or a fistulous hole or opening artificially formed for medical treatment cannot be controlled by the human body's own natural functions; and therefore, ordinarily a body wastes receving pouch appliance is secured to or over the body wastes discharge opening or fistulous opening (hereinafter referred to simply as body wastes discharge opening) to receive the body wastes naturally discharged therefrom, so that the patient can pass his daily life smoothly.

This body wastes receiving pouch appliance is kept in a hermetically sealed state from the outside during use, so that, if only a pouch member thereof is made to contain a deodorant (such as, e.g. polyvinylidene chloride) as a material thereof, the odor of the body wastes received is not allowed to leak out even if the body wastes receiving pouch appliance is used in the state in which the thus received body wastes are stored in said pouch member, but, as soon as the pouch member is released from the body wastes discharge hole or opening of the human body in order to remove the excrements from within the pouch member, the odor leaks from the opening portion of said pouch member, thus causing the people around as well as the patient himself feed bad. In order to avoid this, it may be considered to use a deodorant when the body wastes are taken out for removal from within the pouch member, but it is not possible to prevent the odor from leaking out when the pouch member is released. Further, it is considered to use the method of previously putting a deodorant in the receiving pouch member, but, in this case, the deodorizing action of said deodorant starts to take effect from the point of time when the deodorant is put into the receiving pouch member regardless of whether body wastes are present in the receiving pouch member or not; and thus, this method is very uneconomical. Further, the duration of the deodorizing effect of said deodorant has its limit, and it is very unsanitary and troublesome to remove only the body wastes, leaving the still effective deodorant inside the receiving pouch member or to insert a new deodorant into the receiving pouch member after the removal of only the body wastes from within said receiving pouch member which is still kept in the state secured to the patient's body.

### [Disclosure of the Invention]

It is the object of the present invention to prevent an odor from being produced when the body wastes are removed from a body wastes receiving pouch appliance.

In order to give a solution to the above-mentioned problem, the body wastes receiving pouch appliance according to the present invention comprises a pouch member detachably secured to or over a body wastes discharge hole or opening of a human body so that the body wastes discharged can be received in said pouch member, wherein a hermetically sealed deodorant-containing body is provided, so that, by breaking said deodorant-containing body, the deodorant contained in said deodorant-containing body can be discharged into the pouch member.

It is also possible to mount the deodorant-containing body on the inner side of the pouch member of the body wastes receiving pouch appliance, so that a pressure is applied to said deodorant-containing body from outside the pouch member, whereby said deodorant-containing body is broken to discharge the deodorant into the pouch member from within the deodorant-containing body.

It is alternatively possible to constitute the body wastes receiving pouch appliance in such a manner that the deodortant-containing body is mounted on the outer side of the pouch member of the body wastes receiving pouch appliance, and a hole is formed in that portion of the pouch member which is opposed to the deodorant-containing body, so that, by applying a pressure to the deodorant-containing body, the deodorant-containing body is broken to discharge the deodorant enclosed in the deodorant-containing body into the pouch member through said opposed hole of the pouch member.

It is still alternatively possible to constitute the body wastes receiving pouch appliance in such a manner that, on the pouch member of the body wastes receiving pouch appliance, the deodorant-containing body and a rubbery sealing member are mounted so as to face each other, wherein said rubbery sealing member has an airtightly re-sealing function, so that a needle is thrust into the deodorant-containing body through the rubbery sealing member, whereby the deodorant contained in the deodorant-containing body is discharged into the pouch member, and, after the needle is removed, the rubbery sealing member hermetically seals the pouch member again.

The body wastes receiving pouch appliance may still alternatively be constituted in such a manner that the deodorant-containing body is mounted on the inner side of an adhesive plate of the pouch member of the body wastes receiving pouch appliance, so that a pressure is applied to the deodorant-containing body from outside of the pouch member to break said deodorant-containing body, whereby the deodorant sealed up in the hermetically sealed deodorant-containing body is discharge into the pouch member.

The hermetically sealed deodorant-containing body which may be provided on the inner side or the outer side of the pouch member or the inner side of the adhesive plate of the body wastes receiving pouch appliance is not limited to one in number, but a plurality of such deodorant-containing bodies can be provided. Said deodorant-containing body can be mounted by means of solvent welding, adhesion or the like. Further, the deodorant-containing body can be mounted at the stage of manufacturing the body wastes receiving pouch appliance, but can alternatively be formed separately from the receiving pouch appliance at the manufacutirng stage, so that, later at the time of actual use thereof, the deodorant-containing body can be mounted on the pouch member or on the adhesive plate by means of adhesion or the like. Further, the deodorant-containing body may not be fixed to the pouch member but can be adapted to be thrown into the interior of the pouch member. The breaking of the deodorant-containing body is not limited to the breaking thereof by the application of a manual force, but it is alternatively possible to use, as the packing material of the deodorant-containing body, such a water-soluble material as is not dissolved by the deodorant but the water contained in the body wastes so that said deodorant-containing body may be dissolved by the water content of the body wastes coming into the pouch member.

### [Brief Description of the Drawings]

Fig. 1 shows an embodiment of the present invention, in which a) is a front view thereof, and b) is a longitudinal sectional side view thereof.

Fig. 2 shows a further embodiment of the present invention, in which a) is a front view thereof, and b) is a longitudinal sectional side view thereof.

Fig. 3 shows still further an embodiment of the present invention, in which a) is a front view thereof, b) is a longitudinal sectional side view thereof, and c) is a diagram explaining how the body wastes receiving pouch appliance according to said embodiment is used.

Fig. 4 shows still further an embodiment of the present invention, in which a) is a front view thereof, and b) is a longitudinal sectional side view thereof.

Fig. 5 shows still further an embodiment of the present invention, in which a) is a sectional view thereof, and b) is a front view thereof.

Fig. 6 shows still further an embodiment of the present invention, in which a) is a sectional view thereof, and b) is a front view thereof.

Fig. 7 shows still further an embodiment of the present invention, in which a) is a sectional view thereof, and b), c) and d) are schematic diagrams explaining how this embodiment is used.

Fig. 8 shows still further an embodiment of the present invention, in which a) is a sectional view thereof, and b) is a sectional view showing the body wastes receiving pouch appliance of this embodiment under actual use.

Fig. 9 shows still further an embodiment of the present invention, in which a) and b) are sectional views of the important portion thereof, and c) is an overall conceptual diagram thereof.

Fig. 10 shows still further an embodiment of the present invention, in which a) is a front view of the important portion thereof, b) is a sectional view taken along the line B-B in a), c) is a front view of the important portion thereof, and d) is a sectional view taken along the line D-D in c).

Fig. 11 shows still further an embodiment of the present invention, in which a) and b) are sectional views of the important portion thereof, and c) is an overall conceptual diagram thereof.

Fig. 12 shows still further an embodiment of the present invention, in which a) is a front view thereof, b) is a sectional view thereof, and c) is a schematic diagram explaining this embodiment under actual use.

### [Optimum Embodiments of the Invention]

Embodiments of the present invention will now be described by reference to the drawings.

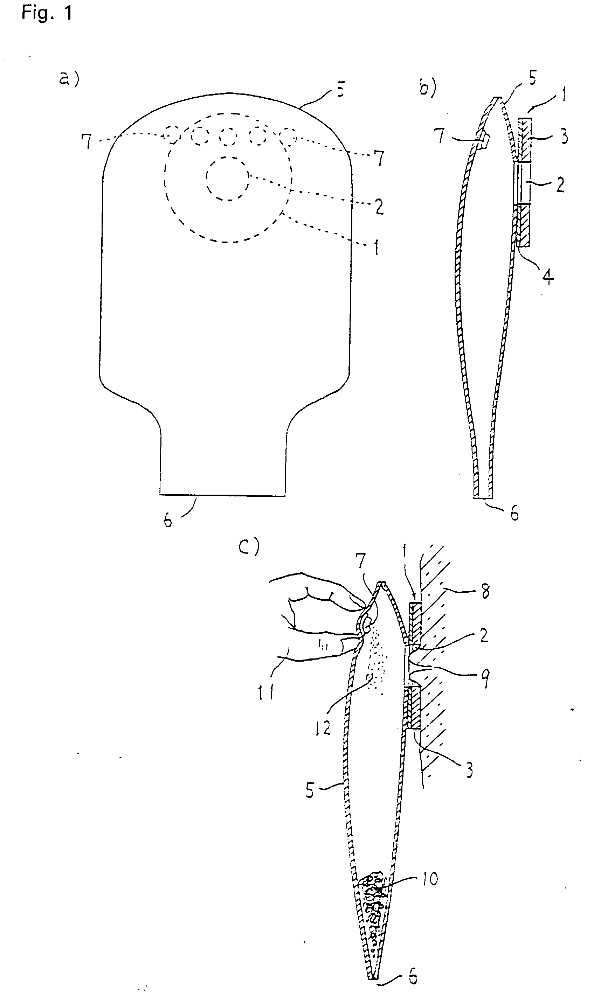

In Fig. 1, a) is a front view of an embodiment of the present invention, b) is a longitudinal sectional side view of said embodiment, and c) is a schematic diagram explaining the manual operation performed for discharging the deodorant into the pouch member. The reference numeral 1 denotes an adhesive plate which is, for instance, in a disk-like shape and has a central opening 2 corresponding to a body wastes discharge hole or opening of a human body to or over which opening the body wastes receiving pouch appliance according to this embodiment is to be secured,. At the adhesive side of said adhesive plate 1, an adhesive layer 3 is provided, while on the surface thereof at the non-adhesive side - that is, the opposite side with reference to said adhesive side - thereof (of said adhesive plate 1), a laminated film 4 is provided. Numeral 5 denotes a pouch member coupled to the peripheral area surrounding the opening 2 at the non-adhesive side of the adhesive plate 1. Said pouch member 5 functions as a container for receiving and storing the body wastes discharged through the opening 2 of the adhesive plate 1 from the body wastes discharge hole or opening of the human body, and a lower end 6 of said pouch member is open for allowing the removal of the body wastes from within the pouch member but can be closed when used. The pouch member 5 is made of a plastics film. Suited as the material of the pouch member 5 is a plastics film which should desirably be water-insoluble and have excellent gas barrier properties, flexibility and sealing properties. As said plastics film, the plastics films used for the formation of the pouch members of the conventional body wastes receiving pouch appliances can also be used; more specifically, there can be pointed out a simple film prepared from, for instance, polyvinylidene chloride, polyethylene, polyvinyl chloride or chlorinated polyehthylene or a composite film obtained by blening or copolymerizing the same with vinyl acetate, polyacrylic acid, or the like.

Numeral 7 denotes a hermetically sealed deodorant-containing body mounted on the inner side of the pouch member 5. Said deodorant-containing body can be constituted in such a manner that a deodorant is wrapped up in a packing material, and the thus formed deodorantcontaining body is fixed to the inner wall of the pouch member 5. This packing material for packing the deodorant must have a strength sufficient to be free from breakage so long as the body wastes receiving pouch appliance is normally handled but can be broken by touching it with fingers or the like. As such a material, for instance polyethylene, EVA (ethylene-vinyl acetate copolymer) or the like is preferable. To the inner wall of the pouch member, the deodorant-containing body can be fixed by means such as, e.g. solvent welding, adhesion or the like. As the deodorant, either the liquid type or the powder type can be used. As liquid type deodorants, the following ones can be pointed out by way of example: "Super Vanish" (trade name) manufactured by Smith & Nephew Inc., U.S.A., "M9" (trade name) manufactured by Sandack Inc., Denmark, "Charm Air" (trade name) manufactured by Eipack Corp., Japan, etc., while, as powder type deodorants, there can be pointed out "Celler Filter" (trade name) manufactured by Nipro Corp., Japan, etc. In the case of the embodiment shown, five hermetically sealed deodorant-containing bodies 7 are provided, but the number of said deodorant-containing bodies 7 is optionally determined. The mounting position of the respective deodorant-containing body can also be optionally determined, but, since the body wastes gather in the lower portion of the pouch member, the deodorant-containing body should desirably be mounted in the upper portion of said pouch member so that the deodorant may be scattered from above, which gives a better deodorizing effect.

Next, the way of using the body wastes receiving pouch appliance according to the present invention will be described. Referring to Fig. 1 c), the body wastes receiving pouch appliance is secured to or over a body waste discharge hole or opening 9 of a human body 8 by the adhesive layer 3 of the adhesive plate 1 thereof, and the lower end 6 is closed up. If it is now assumed that body wastes 10 have gathered in the pouch member 5, so that the body wastes must be taken out for removal or the body wastes receiving pouch appliance must be replaced by a new one. Then, the portion of the pouch membe 5 in which the deodortant-containing body 7 is located is nipped by fingers from outside to apply a pressure to the deodorant-containing body 7, whereby the deodorant-containing body 7 is broken to discharge a deodorant 12 from within the pouch member 5, and thus, a deodorizing action is effected to almost completely eliminate the odor of the body wastes. At this point of time, the adhesive plate 1 is released from the body wastes discharge hole or opening 9, as a resuslt of which it never happens that the odor spreads. In the case where a plurality of such deodorant-containing bodies 7 are provided, the number of deodorant-containing bodies to be broken can be selected depending on the amount of the body wastes present.

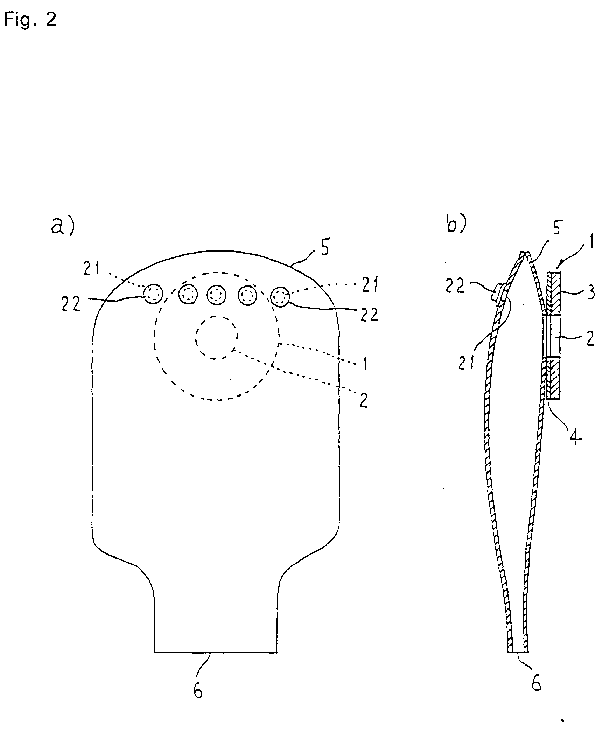

Fig. 2 shows a further embodiment of the present invention, in which a) is a front view thereof, and b) is a longitudinal sectional side view thereof, wherein the same portions as those shown in Fig. 1 are referenced by the same reference numerals. In this embodiment, holes 21 are provided in the pouch member 5, and a deodorant-containing body 22 is mounted on the outer wall of the pouch member 5 in a state opposed to each of said holes 21 so as to cover it. These deodorant-containing bodies 22 are each formed likewise by covering a deodorant with a packing material, in which case a part of each of said deodorant-containing bodies also constitutes the corresponding part of the outer wall of the pouch member 5, so that the outer part of each deodortant-containing body 22 must be composed of a material similar to that of the pouch member which is not broken even if a pressure is applied thereto, while the part of said each deodorant-containing body which is opposed to the hole 21 must be composed of a material which is readily broken by the application of such a pressure. For instance, the blister packaging type is suited. As for the packing material in this case, the outer packing or covering portion of each deodorant-containing body can be composed of for instance vinyl chloride, polyester resin, or the like, while the inner packing or covering portion thereof can be composed of for instance an aluminum/polyethylene-laminated film, wherein the inner-side polyethylene layer serves as a layer joining to the pouch member and can be fixed, by solvent welding, to the peripheral area around each hole 21 of the pouch member 5. The way of using this body wastes receiving pouch appliance is similar to that of the foregoing embodiment shown in Fig. 1 but this embodiment is advantageous in that the operation of breaking the deodorant-containing bodies is relatively easy since the deodorant-containing bodies are somewhat projected outwardly.

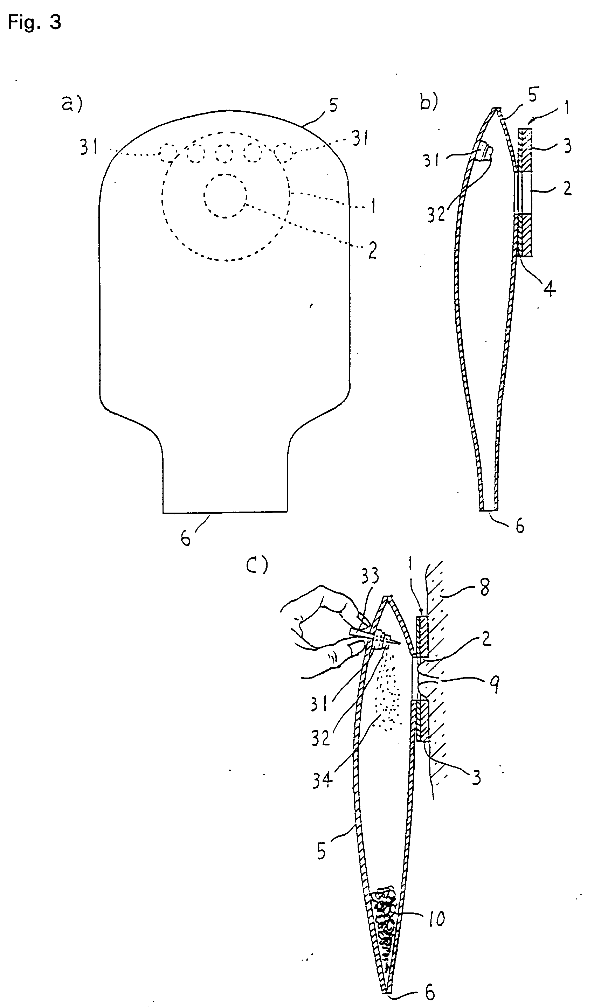

Fig. 3 shows still further an embodiment of the present invention, in which a) is a front view of said embodiment, b) is a longitudinal sectional side view thereof, and c) is a schematic diagram explaining the way of using the body wastes receiving pouch member according to this embodiment, wherein the same portions as those shown in Fig. 1 are referenced by the same reference numerals. In this embodiment, rubbery sealing members 31 are mounted on the inner wall of the pouch member 5, and, on these rubbery sealing members 31, deodorant-containing bodies 32 are mounted, respectively. The rubbery sealing members 31 must be composed of such a material as allows a needle to be thrust therethrough when the deodorant is to be discharged into the pouch member from within each deodorant-containing body 32, but on the other hand, can hermetically re-seal the pouch member after the needle is drawn out, leaving no trace of the hole made by the thrust needle. Such a function can be imparted to the rubbery selaing members by composing them of, for instance, CR (chloroprene rubber), acrylic rubber, chlorinated rubber, ethylene/propylene rubber, silicone rubber, NBR (nitrile rubber), SBR (styrene-butadiene rubber), urethane rubber, or the like. The deodorant-containing bodies 32 are composed by hermetically sealing up a deodorant in a packing material as in the case of the respective foregoing embodiments, wherein the packing material thus wrapping the deodorant up is ready to be broken by thrusting a needle therethrough. Usable as the packing material are, for instance, polyethylene, EVA (ethylene-vinyl acetate copolymer), vinyl chloride and polypropylene. The thus formed deodorant-containing bodies 32 are fixed to the rubbery sealing members 31 by means such as for instance solvent welding, adhesion, or the like. As for the way of using the body wastes receiving pouch appliance according to this embodiment, a needle 33 is thrust through the rubbery sealing member 31 and the deodorant-containing body 32 to break the seal of the deodorant-containing body 32, whereby the deodorant 34 is discharged.

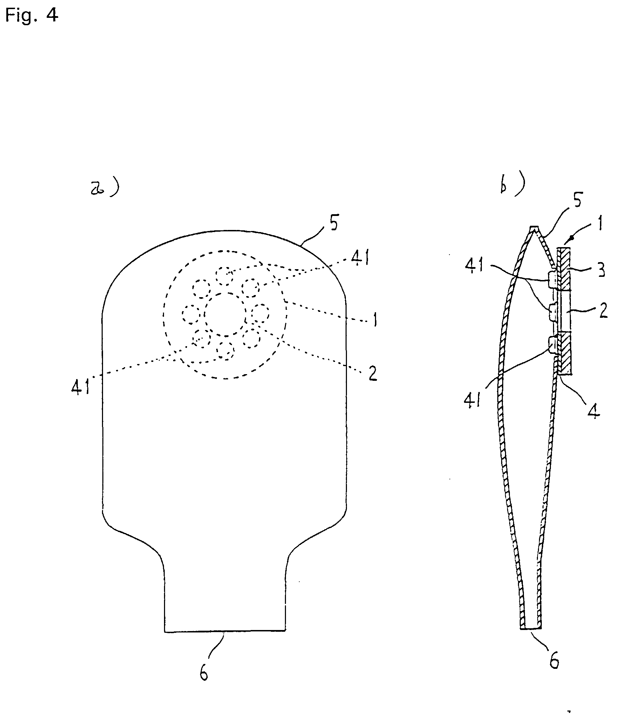

Fig. 4 shows still further an embodiment of the present invention, in which a) is a front view of said embodiment, and b) is a longitudinal sectional side view thereof, wherein the same portions as those shown in Fig. 1 are referenced by the same refernce numerals. In this embodiment, hermetically sealed deodorant-containing bodies 41 are mounted on the portion of the adhesive plate 1 which faces the interior of the pouch member, that is, on the portion of the laminated film 4 of the adhesive plate which portion lies at the inner side of the pouch member. In the case of the embodiment shown, a plurality of deodorant-containing bodies are provided in a state surrounding the opening 2. The deodorant-containing bodies 41 each can be formed by wrapping up a deodorant in a packing material and fixed to the adhesive plate by solvent welding or adhesion as in the case of the embodiment shown in Fig. 1. As for the way of using the body wastes receiving pouch applicance according to this embodiment, a pressure is applied to each deodorant-containing body 41 from the outer side of the pouch member 5 to break said deodorant-containing body 41, whereby the deodorant is discharged into the pouch member, as in the case of the embodiment shown in Fig. 1.

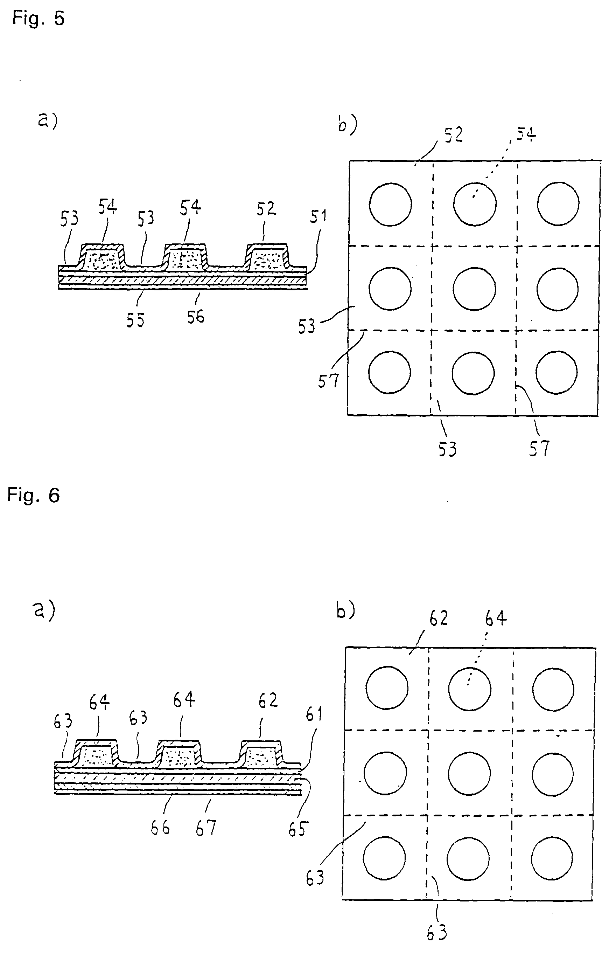

Fig. 5 shows an exemplary concrete constitution of hermetically sealed deodorant-containing bodies suited for use in the case where the deodorant-containing bodies are not mounted until the patient puts on the body wastes receiving pouch applicance by himself or the surgical operator puts it on the patient. In Fig. 5, a) is a longitudinal sectional side view, and b) is a plan view. The reference numeral 51 denotes a base material. To the surface of said base material 51, a packing material 52 is partially solvent-welded (53), whereby a deodorant 54 is sealed up inside the packing material 52. Onto the other side of the base material 51, an adhesive layer 55 is attached, and said adhesive layer 55 is covered by a release paper 56. On the base material 51, a plurality of individual deodorant-containing bodies can be provided in the strip packaging manner, in which case perforations 57 are previously provided in the packing material as may be apparent from Fig. 5b), so that a necessary number of deodorant-containing bodies can be torn off for each use. As the base material, a mono- or double-layer film with a solvent-weldable layer made of a material such as, e.g. polyethylene, polypropylene, ehtylene-vinyl acetate copolymer, or the like is used, and, as the packing material, any of the substances pointed out in connection with the embodiment shown in Fig. 1 can be used. As for the deodorant-containing bodies, it is not always necessary to provide a plurality of them on the base material, but it is possible to provide only one such deodorant-containing body, instead. Said deodorant-containing bodies are each fixed to a suitable location inside the pouch member by means of the adhesive layer 55 exposed by removing the release paper 56.

Fig. 6 shows another embodiment of the concrete structure of the deodorant-containing bodies suited for use in the case where they are not mounted on the pouch member until the body wastes receiving pouch appliance is not put on the patient by the patient himself or by the surgical operator as in the case of the embodiment shown in Fig. 5. In Fig. 6, a) is a longitudinal sectional side view, and b) is a plan view. Numeral 61 denotes a base material, onto the surface of which a packing material 62 is partially solvent-welded (63), whereby a deodorant 64 is hermetically sealed up inside said packing material 62 as in the case of the embodiment shown in Fig. 5, but, in the case of this embodiment, on the opposite side of the base material 61, a rubbery sealing material 65, an adhesive layer 66, and a release paper 67 are provided. In this case, also, either a single deodorant-containing body or a plurality of deodorant-containing bodies formed in succession may be provided. The deodorant-containing bodies in this embodiment are of the type that the packing material of each deodorant-containing body is broken by thrusting a needle therethrough as in the case of the embodiment shown in Fig. 3; and the rubbery sealing members can be composed of the same material as that used in the embodiment shown in Fig. 3. In this case, also, a susitable number of deodorant-containing bodies are fixed to the inner side of the pouch member by means of an adhesive layer 66 exposed after removal of the release paper 67.

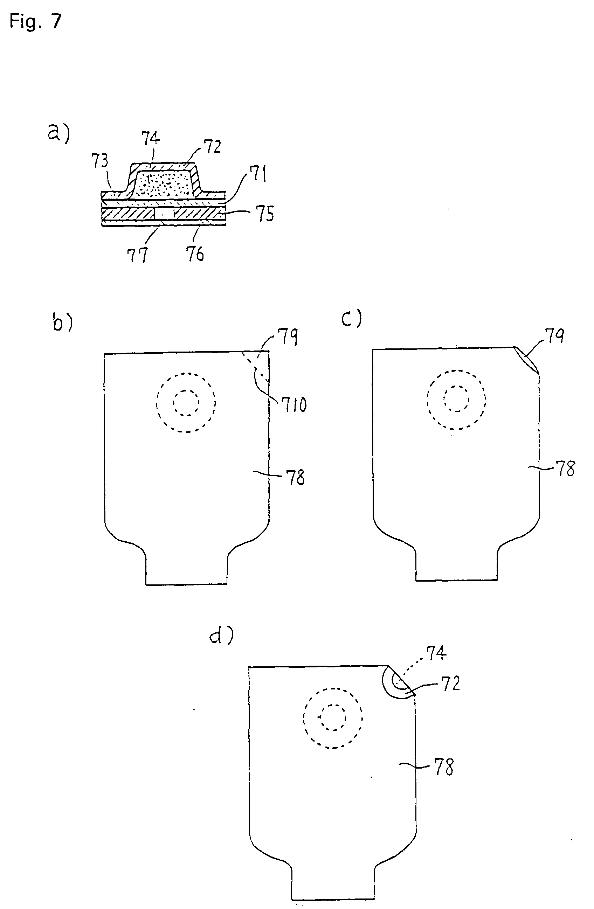

Fig. 7 shows still further an embodiment of the concrete structure of the deodorant-containing body suited for use in the case where the deodorant-containing body is not mounted on the pouch member until the patient himself or the surgical operator puts the body wastes receiving pouch appliance on the patient as in the case of the embodiment shown in Fig. 5. In Fig. 7, a) is a longitudinal sectional view, and b), c) and d) are plan views used for explaining the way of using this embodiment. Numeral 71 denotes a base material, to the surface of which a packing material 72 is partially solvlent-welded (73), whereby a deodorant 74 is enclosed inside thereof, and, on the opposite side of the base material 71, an adhesive layer 75 and a release paper 76 are provided. In this embodiment, the packing material 72 has such a strength that it is not broken even if a pressure is applied thereto unlike the embodiment shown in Fig. 5, while the base material 71 is composed of a material which can be broken by the application of a pressure thereto. Further, in the center portion of the adhesive layer 75, a throughhole 77 is provided, so that, when a rpessure is applied to the packing material 72 to break the base material 71, the deodorant 74 can flow out through this throughhole 77. This deodorant-containing body is used in such a manner that, immediately before the actual use thereof, a portion of the pouch member of the body wastes receiving pouch appliance is cut off, and, to the portion thus cut off, said deodorant-containing body is attached by means of the adhesive layer 75. That is, as shown in Fig. 7b, a corner 79 of the pouch member 78 is cut off along a broken line 710, the thus cut corner 79 is then opened as shown in Fig. 7c), and the deodorant-containing body is folded in two with the adhesive layer 75 turned inside and then fixed by means of said adhesive layer 75 in a state extending over the surface portions, at both sides, of the corner 79 of the pouch member 78. In this case, the position of the pouch member at which the deodorant-containing body is fixed is optionally selectable.

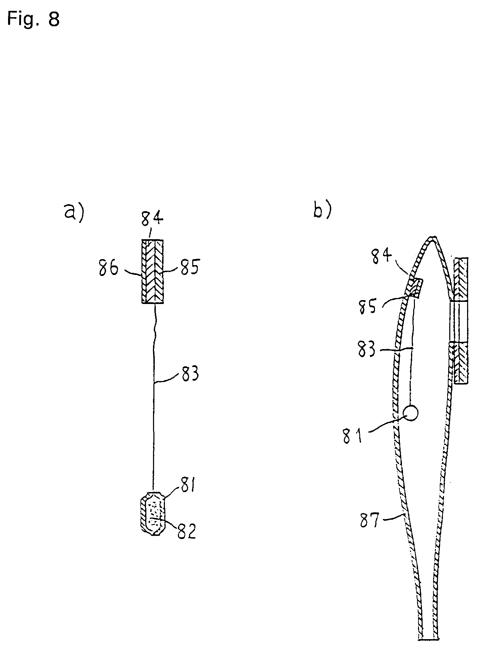

Fig. 8 shows an embodiment adapted in such a manner that the deodorant-containing body is indirectly fixed to the pouch member. In Fig. 8, a) is a sectional view of the deodorant-containing body, and b) is a sectional view of the body wastes receiving pouch appliance with its deodorant-containing body mounted thereon. Numeral 81 denotes a packing material, inside of which a deodorant 82 is enclosed. To the packing material 81, a thread-like member 83 such as for instance a thread or a filament is coupled, and, to said thread-like member 83, a laminated body consisting of an adhesive layer 84 and a coating layer 85 is coupled. Further, the adhesive layer 84 is covered with a release paper 86. This deodorant-containing body can be inserted into a pouch member 87 through an opening thereof before the actual use of the body wastes receiving pouch appliance and fixed to the inner wall of said pouch member 87 by the adhesive layer 84. Said deodorant-containing body is broken by application of a pressure thereto so as to discharge the deodorant, but, in this case, the deodorant-containing body is freely movable by means of the thread-like member 83 during use of the body wastes receiving pouch appliance, so that said deodorant-containing body is prevented from being subjected to any undue force.

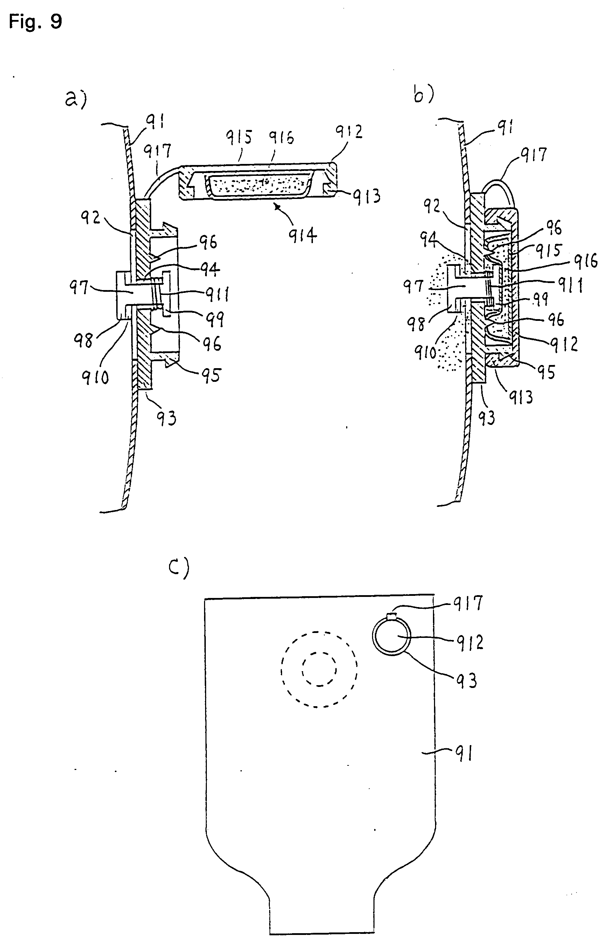

Fig. 9 shows an embodiment of the body wastes receiving pouch appliance according to the present invention which is constituted in such a manner that, immediately before the body wastes in the body wastes receiving pouch appliance are disposed of, a deodorant can be inserted into the pouch member. In Fig. 9, a) is a longitudinal sectional view showing the state of the important portion of this embodiment before the body wastes therein are disposed of, b) is a longitudinal sectional view showing the state of the important portion of this embodiment during the diposal of the body wastes, and c) is an overall conceptual diagram of this embodiment. Numeral 91 denotes the pouch member of the body wastes receiving pouch appliance, and numeral 92 denotes an opening provided in a suitable location (in the upper right portion in the case of the embodiment shown) of the pouch member 91. Numeral 93 denotes an inner cover of a cartridge for mounting the deodorant-containing body therein; said inner cover 93 is fixed, in its outer peripheral portion, to or around the opening 92 of the pouch member 91 by means of solvent welding or the like. Said inner cover 93 has a central opening 94 corresponding to the opening 92 of the pouch member 91, a male type fitting portion 95 in the vicinity of the outer periphery thereof at the opposite side with reference to the pouch member 91, and a plurality of porjections 96 inside said fitting portion 95. Into the opening 94 of the inner cover 93, a plug 97 is fitted, and, at the tip end, on the pouch-member (91) side, of said plug 97, a flange 98 is provided, while on the opposite side, a flange 99 is provided. Further, on the side of said flange 98 which is opposed to the inner cover 93, a packing ring 910 is inserted, while on the side of the flange 99 which is opposed to the inner cover 93, a spring 911 is fitted. Numeral 912 denotes an outer cover of the cartridge. Said outer cover 912 has, in its outer peripheral portion, a female type fitting portion 913 which can be engaged with the male type fitting portion 95 of the inner cover 93, and, inside of said outer cover 912, a deodorant-containing body 914 can be inserted. The deodorant-containing body 914 comprises a deodorant 916 sealed up in a packing material 915. The inner cover 93 and the outer cover 912 are coupled to each other by means of a member 917. In the state before the body wastes are disposed of by the deodorant, the inner cover 93 and the outer cover 912 of the cartridge are separated from each other, wherein the flange 98 is pushed against the rear side of the inner cover 93 by the spring action of the spring 911, and thus, the opening 94 of the inner cover 93 is shut off from the outside by the packing 910. At the time of disposing of the body wastes, the outer cover 912 is fitted to the inner cover 93 as shown in Fig. 9b) and the outer cover 912 is pressed from outside, whereby a packing material 915 is broken by the projections 96, while the plug 97 is moved toward the interior of the pouch member 91 against the spring all, so that the deodorant 916 is discharged into the pouch member 91 through the openings 94 and 92. Further, it is alternatively possible to constitute the body wastes receiving pouch appliance in such a manner that, in the state in which the body wastes are not disposed of, the inner cover 93 and the outer cover 912 are not kept in a separated state, that is, they are fitted to each other without inserting the deodorant-containing body 914 into the outer cover 912, so that, at the stage of disposing of the body wastes, the two covers 93 and 912 are separated to open, the deodorant-containing body 914 is inserted into the outer cover 912, and then the two covers are closed again.

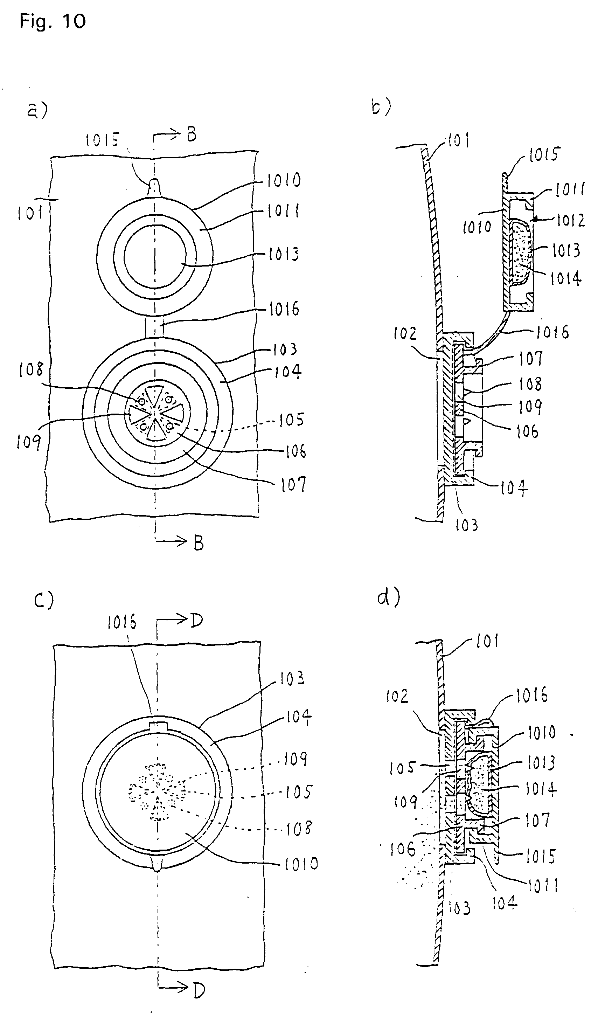

Fig. 10 shows a different embodiment of the body wastes receiving pouch appliance which embodiment is constituted in such a manner that, immediately before the disposal of the body wastes in the body wastes receiving pouch appliance, the deodorant can be inserted into the pouch member as in the case of the embodiment shown in Fig. 9. In Fig. 10, a) is a front view showing the state of the important portion of the body wastes receiving pouch appliance before the disposal of the body wastes, b) is a sectional view taken along the line B-B in a), c) is a front view of the important portion of the body wastes receiving pouch appliance during disposal of the body wastes, and d) is a sectional view taken along the line D-D in c). Numeral 101 denotes the pouch member of the body wastes receiving pouch appliance. Numeral 102 denotes an opening provided in a suitable portion of said pouch member 101. Numeral 103 denotes a disk-shaped fixed frame of a cartridge for mounting a deodorant-containing body therein. Said fixed frame 103 is fixed to the peripheral area around the opening 102 by sovlent welding or the like, and the outer peripheral portion thereof constitutes a flange 104 which is L-shaped in section, and throughholes - four fan-shaped throughholes 105 in the case of the embodiemnt shown - are provided in that portion of the fixed frame 103 which is opposed to the opening 102. Numeral 106 is a disk-shaped inner cover, which is housed in the flange 104 of the fixed frame 103 and held so as to be rotatable in the fixed frame 103. The inner cover 106 has a male type fitting portion 107 in the vicinity of the outer periphery thereof. Said inner cover 106 also has projections 108 and fan-shaped throughholes 109 in the inner side of said male type fitting portion 107. Numeral 1010 denotes an outer cover, which has, in its outer peripheral portion, a female type fitting poriton 1011 which can be engaged with the male type fitting portion 107 of the inner cover 106, and, into the inside of said female type fitting portion 1011, a deodorant-containing body 1012 can be inserted. The deodorant-containing body 1012 comprises a deodorant 1014 sealed up in a packing material 1013. The outer cover 1010 has a manipulating handle 1015. The inner cover 106 and the outer cover 1010 are coupled to each other by a coupling member 1016. In the state before the body wastes are disposed of, the inner cover 106 and the outer cover 1010 of the cartridge are kept in a state separted from each other, and the throughholes 105 of the fixed frame 103 and the throughholes 109 of the inner cover 106 are displaced from each other as may be understood from Fig. 10a), and thus, the space in the pouch member 101 and the outside are shut off from each other. When the body wastes are to be disposed of, the deodorant-containing body 1012 is inserted into the outer cover 1010, the outer cover 1010 and the inner cover 106 are fitted to each other, and then the outer cover 1010 is pressed from outside, whereby the packing material 1013 is broken by the projections 108. Then, the inner cover 106 and the outer cover 1010 which are now brought into one integral body is rotated, by means of the handle 1015, within the flange 104 to align the throughholes 109 of the inner cover 106 with the throughholes 105 of the fixed frame 103 as shown in Fig. 10c) and Fig. 10d), whereby, from the broken packing material 1013, the deodorant 1014 is discharged into the pouch member 101 via the throughholes 109 and 106, and the opening 102 of the pouch member 101. Further, it is alternatively possible to constitute the body wastes receiving pouch appliance in such a manner that, in the state in which the body wastes are not disposed of, the inner cover 106 and the outer cover 1010 are not separated from each other, that is, they are fitted to each other with the deodorant-containing body 1012 not inserted into the outer cover 1010, as in the case of the embodiment shown in Fig. 9, so that, at the stage of disposing of the body wastes, the two covers 106 and 1010 are separated to open, and the deodorant-containing body 1012 is inserted, and then, the two covers are closed again.

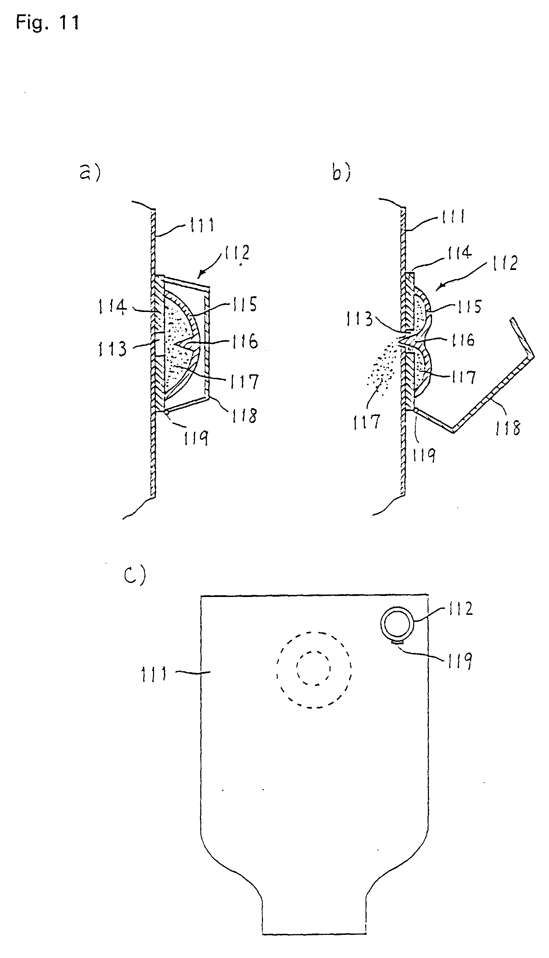

Fig. 11 shows still different an embodiment of the body wastes receiving pouch appliance of the type which is constituted in such a manner that, immediately before the disposal of the body wastes in the body wastes receiving pouch appliance, the deodorant can be inserted into the pouch member as in the case of the embodiment shown in Fig. 9. In Fig. 11, a) is a longitudinal sectional view showing the state of the important portion of the body wastes receiving pouch appliance before disposal of the body wastes, b) is a longitudinal sectional view of the body wastes receiving pouch appliance during disposal of the body wastes, and c) is an overall conceptual diagram of the body wastes receiving pouch appliance. Numeral 111 denotes a pouch member of the body wastes receiving pouch appliance, and numeral 112 denotes a deodorant-containing body mounted at a suitable location (in the upper right portion in the case of the embodiment shown) of the pouch member 111. The deodorant-containing body 112 is constituted in such a manner that, to the surface of an ahesive layer 114 which has a throughhole 113 in the center portion thereof, a deodorant-filled vessel 115 is mounted. This deodorant-filled vessel 115 has therein a projection 116 projecting towards the throughhole 113 of the adhesive layer 114 and has a deodorant 117 sealed up therein. Further, to said adhesive layer 114, a hard case 118 is hinge-coupled (119) so as to cover the deodorant-filled vessel 115. The deodorant-containing body 112 is fixed to the outer surface of the pouch member 111 by means of the adhesive layer 114 thereof. This body wastes receiving pouch appliance is ordinarily used with the hard case 118 closed. When body wastes have been discharged into the pouch member 111, and thus, it becomes necessary to dispose of said body wastes, the hard case 118 is opened by manually turning it about the hinge coupler 119, and the deodorant-filled vessel 115 is pushed by the hand, whereby the vessel 115 is deformed, so that the projection 116 is moved toward the pouch member 111 till the tip end of said projection 116 breaks the pouch member 111. Therefore, the deodorant 117 is discharged into the pouch member 111 via the throughhole 113 of the adhesive layer 114.

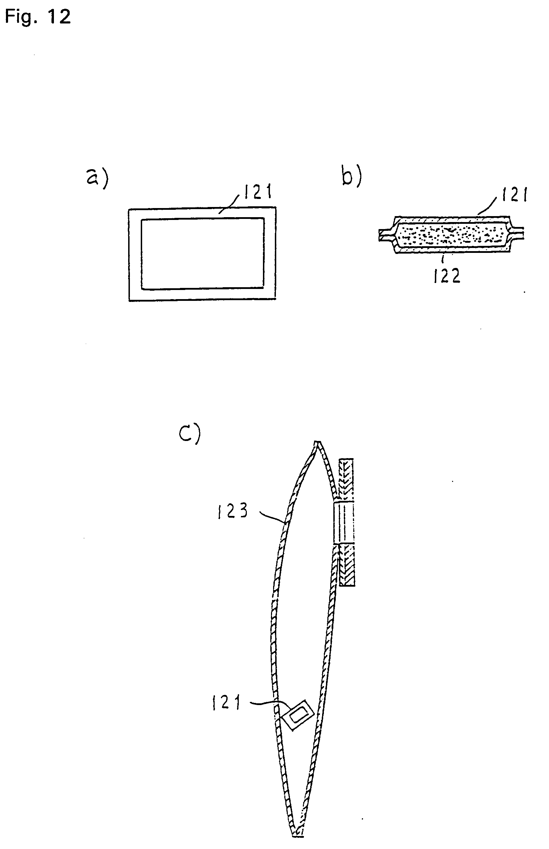

Fig. 12 shows still different an embodiment of the present invention which is constituted in such a manner that the deodorant-containing body need not be fixed to the inner surface or the outer surface of the pouch member of the body wastes receiving pouch appliance. In Fig. 12, a) is a plan view of said embodiment, b) is a longitudinal sectional view thereof, and c) is a schematic diagram explaining how the body wastes receiving pouch appliance according to this embodiment is used. Numeral 121 denotes a packing container, in which a deodorant 122 is sealed up. In this case, a required number of deodorant-containing bodies are put into a pouch member 123 prior to the actual use of the body wastes receiving pouch appliance. The packing container 121 can be composed of such a material as can be broken by application of a pressure. As the material thereof, the substances pointed out above in connection with the embodiment shown in Fig. 1 can also be used. Further, if the packing container 121 is formed of a water-soluble film, then said packing container 121 can be broken without applying any particular pressure thereto since it is dissolved by the water contained in the body wastes. As the material of the water-soluble film, for instance polyvinyl alcohol can be used. In this case, however, the deodorant must comprise a perfectly hydrophobic substance; if otherwise, there is the fear that the water-soluble film may be dissolved by the deodorant before body wastes are discharged into the pouch member. As hydrophobic deodorants, active carbon, ceramics, ion exchange resins, active alumina, zeolite, etc. can be used.

In the embodiments described above, the body wastes receiving pouch appliances are of the so-called one-piece type in which the pouch member and the adhesive plate are not separated, but the present application can also be applied to the so-called two-piece type in which the pouch member is detachably secured to the adhesive plate. Further, as the pouch member, the various conventional pouch members can also be used in the present invention.

According to the present invention, there can be provided body wastes receiving pouch appliances each constituted in such a manner that, immediately before the body wastes are taken out for removal from within the pouch member or immediately before the pouch member is released from the body wates discharge hole or opening of a human body, the deodorant-containing body is broken to discharge the deodorant, whereby the deodorizing effect thereof can be exhibited most effectively, and the dispersion or diffusion of the odor at the time of releasing the pouch member can be surely prevented; the deodorant is previously mounted, so that it is not ncessary to do the troublesome work of inserting the deodorant later; and it is also very easy to mount the deodorant-containing body on the pouch member immediately before the body wastes are taken out for removal or immediately before the pouch member is released from the body wastes discharge hole or opening of a human body, and thus, the handling of the body wastes receiving pouch appliance according to the present invention is by far comfortable and easy as compared with the conventional body wastes receiving pouch appliances. In addition, by applying the present invention to the conventional body wastes receiving pouch appliances sold at the market at present, similar effects can also be obtained.

## Claims

1. Body wastes receiving pouch appliance comprising a pouch member which can be detachably secured to or over a body wastes discharge hole or opening of a human body, said pouch member being able to receive and store body wastes therein, characterized by further comprising a hermetically sealed deodorant-containing body constituted in such a manner that, when said deodorant-containing body is broken, the deodorant sealed up in said deodorant-containing body can be discharged into said pouch member.

2. Body wastes receiving pouch appliance according to Claim 1, wherein the deodorant-containing body is mounted on the inner side of the pouch member, so that, by applying a pressure to said deodorant-containing body from outside, said deodorant-containing body can be broken.

3. Body wastes receiving pouch appliance according to Claim 1, wherein the deodorant-containing body is mounted on the outer side of the pouch member, and a hole is provided in that portion of said pouch member which is opposed to said deodorant-containing body, so that, by applying a pressure to said deodorant-containing body, said deodorant-containing body is broken, whereby the deodorant sealed up in said deodorant-containing body is discharged into said pouch member through the opposed hole of said pouch member.

4. Body wastes receiving pouch appliance according to Claim 1, wherein, on the pouch member, the deodorant-containing body and a rubbery sealing member are mounted so as to face each other, said rubbery sealing member possessing a hermetically re-sealing function, so that, by thrusting a needle into said deodorant-containing body through said rubbery sealing member, said deodorant-containing body is broken, and, after said needle is removed, said rubbery sealing member can hermetically seal said pouch member again.

5. Body wastes receiving pouch appliance according to Claim 1, wherein the deodorant-containing body is mounted on the inner side of the adhesive plate of the pouch member, so that, by applying a pressure to said deodorant-containing body from outside, said deodorant-containing body is broken.

6. Body wastes receiving pouch appliance according to Claim 1, wherein the deodorant-containing body has an adhesive layer, so that, by said adhesive layer, said deodorant-containing body can be mounted on the inner side of the pouch member.

7. Body wastes receiving pouch appliance according to Claim 1, wherein the deodorant-containing body is suspended from an adhesive member by means of a thread-like member, so that said deodorant-containing body can be mounted inside of the pouch member by means of said adhesive member.

8. Body wastes receiving pouch appliance according to Claim 1, wherein the deodorant-containing body is formed as a single separate body, which can be arbitrar thrown into the pouch member.

9. Body wastes receiving pouch appliance according to Claim 1, wherein the deodorant-containing body comprises a deodorant sealed up in a water-soluble film, so that said deodorant-containing body can be broken by the water contained in the body wastes.

10. Body wastes receiving pouch appliance according to Claim 1, wherein plural deodorant-containing bodies are provided.
